(19) Europäisches Patentamt
European Patent Office
Office européen des brevets

(11) Veröffentlichungsnummer: **0 124 804**
**A2**

# (12) EUROPÄISCHE PATENTANMELDUNG

(21) Anmeldenummer: **84104276.5**

(22) Anmeldetag: **16.04.84**

(51) Int. Cl.³: **C 07 D 405/12**
A 01 N 47/24

(30) Priorität: **27.04.83 DE 3315262**

(43) Veröffentlichungstag der Anmeldung:
**14.11.84 Patentblatt 84/46**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Heywang, Gerhard, Dr.**
**Nittumer Weg 4**
**D-5060 Bergisch-Gladbach 2(DE)**

(72) Erfinder: **Reinecke, Paul, Dr.**
**Lessingstrasse 11**
**D-5090 Leverkusen 3(DE)**

(72) Erfinder: **Brandes, Wilhelm, Dr.**
**Eichendorffstrasse 3**
**D-5653 Leichlingen 1(DE)**

(72) Erfinder: **Hänssler, Gerd, Dr.**
**Heymannstrasse 40**
**D-5090 Leverkusen 1(DE)**

(72) Erfinder: **Hammann, Ingeborg, Dr.**
**Lutherstrasse 22**
**D-4330 Mülheim/Ruhr(DE)**

## (54) Carbamoylierte Azolylalkyl-phenoxy-carbinole, Verfahren zu ihrer Herstellung und ihre Verwendung als Pflanzenschutzmittel.

(57) Die Erfindung betrifft neue carbamoylierte Azolylalkyl-phenoxy-carbinole, ein Verfahren zu ihrer Herstellung sowie ihre Verwendung als Pflanzenschutzmittel.

Die neuen Verbindungen der Formel

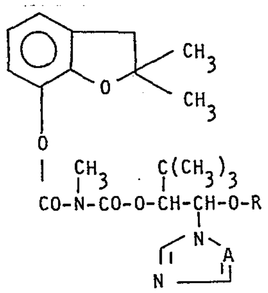

(I)

in welcher A und R die in der Beschreibung angegebene Bedeutung besitzen, werden erhalten, wenn man 1-Azolyl-3,3-dimethyl-1-phenoxy-2-butanole mit N-(2,3-Dihydro-2,2-dimethyl-benzofuran-7-oxy-carbonyl)-N-methyl-carbamidsäurehalogeniden umsetzt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet. Sie zeichnen sich durch eine fungizide und auch durch eine insektizide Wirkung aus.

EP 0 124 804 A2

BAYER AKTIENGESELLSCHAFT 5090 Leverkusen, Bayerwerk
Konzernverwaltung RP
Patentabteilung
Slr/AB
Ia

# Carbamoylierte Azolylalkyl-phenoxy-carbinole, Verfahren zu ihrer Herstellung sowie ihre Verwendung als Pflanzenschutzmittel

Die vorliegende Erfindung betrifft neue carbamoylierte Azolylalkyl-phenoxy-carbinole, ein Verfahren zu ihrer Herstellung sowie ihre Verwendung als Pflanzenschutzmittel.

Es ist bereits bekannt geworden, daß bestimmte carbamoylierte 3,3-Dimethyl-1-phenoxy-1-(1,2,4-triazol-1-yl)-2-butanole, wie beispielsweise 1-(4-Biphenylyloxy)-3,3-dimethyl-2-methoxycarbonylcarbamoyloxy-1-(1,2,4-triazol-1-yl)-butan oder 1-(4-Biphenylyloxy)- bzw. 1-(2,5-Dichlorphenoxy)- bzw. 1-(2,4,5-Trichlorphenoxy)-3,3-dimethyl-2-methylcarbamoyloxy-1-(1,2,4-triazol-1-yl)-butan, gute fungizide Eigenschaften besitzen (vergleiche DE-OS 26 00 799 [LeA 16 838] und DE-OS 28 00 544 [LeA 18 620]). Die Wirkung dieser Verbindungen ist jedoch in bestimmten Indikationsbereichen, insbesondere bei niedrigen Aufwandmengen und -konzentrationen, nicht immer ganz befriedigend.

Es wurden neue carbamoylierte Azolylalkyl-phenoxy-carbinole der allgemeinen Formel

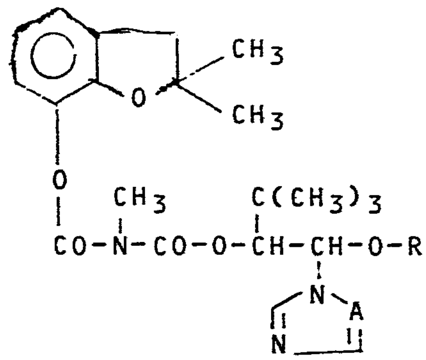

(I)

in welcher

A für ein Stickstoffatom oder die CH-Gruppe steht und

R für gegebenenfalls substituiertes Phenyl steht,

gefunden.

Die Verbindungen der Formel (I) besitzen zwei asymmetrische Kohlenstoffatome; sie können somit in zwei geometrischen Isomeren (threo- und erythro-Form) vorliegen. Vorwiegend fallen sie als Gemische beider Formen an.

Weiterhin wurde gefunden, daß man die carbamoylierten Azolylalkyl-phenoxy-carbinole der Formel (I) erhält, wenn man 1-Azolyl-3,3-dimethyl-1-phenoxy-2-butanole der Formel

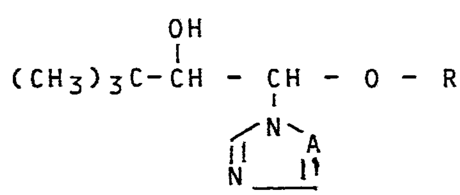

(II)

in welcher

A und R die oben angegebene Bedeutung haben,

mit N-(2,3-Dihydro-2,2-dimethyl-benzofuran-7-oxy-carbonyl)-N-methyl-carbamidsäurehalogeniden der Formel

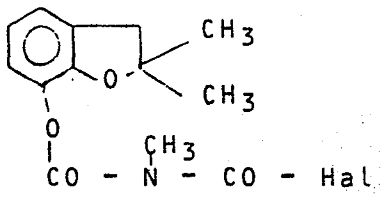

(III)

in welcher

Hal für Halogenid, vorzugsweise Chlor oder Brom steht,

in Gegenwart eines Verdünnungsmittels und in Gegenwart eines Säurebindemittels umsetzt.

An die so erhaltenen Verbindungen der Formel (I) kann gegebenenfalls anschließend noch eine Säure oder ein Metallsalz addiert werden.

Die neuen carbamoylierten Azolylalkyl-phenoxy-carbinole der Formel (I) weisen starke fungizide und insektizide Eigenschaften auf. Dabei zeigen überraschenderweise die erfindungsgemäßen Verbindungen eine bessere fungizide und eine bessere insektizide Wirkung als die aus dem Stand der Technik bekannten carbamoylierten 3,3-Dimethyl-1-phenoxy-1-(1,2,4-triazol-1-yl)-2-butanole, wie 1-(4-Biphenylyloxy)-3,3-dimethyl-2-methoxycarbonylcarbamoyloxy-1-(1,2,4-triazol-1-yl)-butan oder 1-(4-Biphenylyloxy)- bzw. 1-(2,5-Dichlorphenoxy)- bzw. 1-(2,4,5-Trichlorphenoxy)-3,3-dimethyl-2-methylcarbamoyloxy-1-(1,2,4-triazol-1-yl)-butan, welches konstitutionell und wirkungsmäßig naheliegende Verbindungen sind. Die erfindungsgemäßen Stoffe stellen somit eine Bereicherung der Technik dar.

Die erfindungsgemäßen carbamoylierten Azolylalkylphenoxycarbinole sind durch die Formel (I) allgemein definiert. In dieser Formel stehen vorzugsweise

A für ein Stickstoffatom oder die CH-Gruppe und

R für gegebenenfalls einfach oder zweifach, gleich oder verschieden substituiertes Phenyl, wobei als Substituenten genannt seien: Halogen, Alkyl mit 1 bis 4 Kohlenstoffatomen und gegebenenfalls durch Halogen substituiertes Phenyl.

Besonders bevorzugt sind diejenigen Verbindungen der Formel (I), in denen

A für ein Stickstoffatom oder die CH-Gruppe steht und

R für durch Fluor, Chlor, Methyl oder Phenyl substituiertes Phenyl steht.

Ganz besonders bevorzugt sind diejenigen Verbindungen der Formel (I), in denen

A für ein Stickstoffatom steht und

R für durch Fluor, Chlor oder Phenyl substituiertes Phenyl steht.

Verwendet man beispielsweise 1-(4-Chlorphenoxy)-3,3-dimethyl-1-(1,2,4-triazol-1-yl)-2-butanol und N-2,3-Dihydro-2,2-dimethyl-benzofuran-7-oxy-carbonyl)-N-methyl-carbamidsäurechlorid als Ausgangsstoffe, so kann der Reaktionsablauf des erfindungsgemäßen Verfahrens durch das folgende Formelschema wiedergegeben werden:

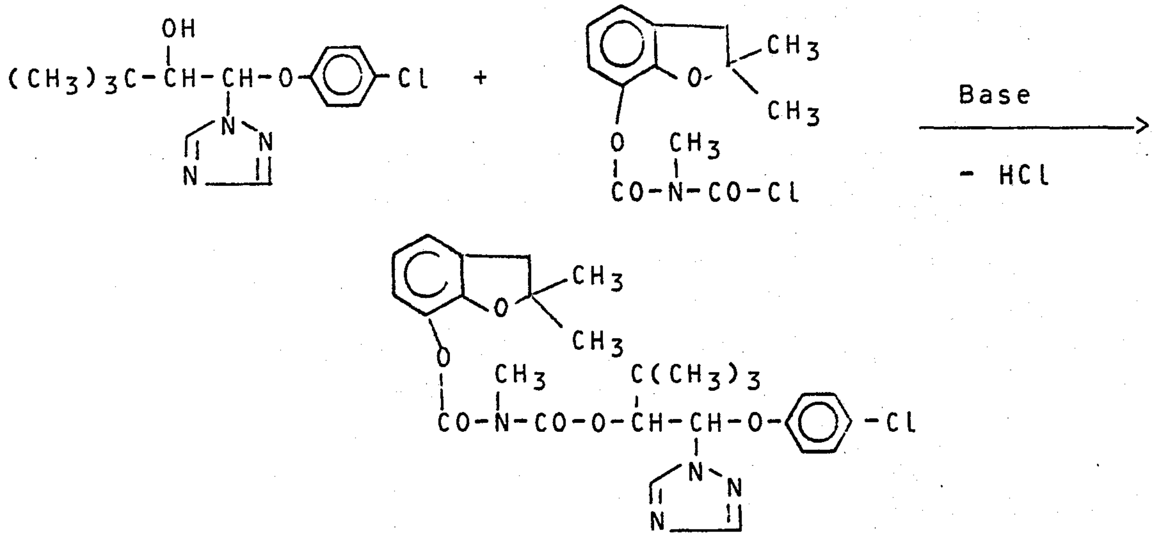


Die bei der Durchführung des erfindungsgemäßen Verfahrens als Ausgangsstoffe benötigten 1-Azolyl-3,3-dimethyl-1-phenoxy-2-butanole sind durch die Formel (II) allgemein definiert. In dieser Formel stehen A und R vorzugsweise für die Bedeutungen, die bei der Beschreibung der erfindungsgemäßen Stoffe der Formel (I) bereits vorzugsweise für diese Reste genannt wurden.

Die 1-Azolyl-3,3-dimethyl-1-phenoxy-2-butanole der Formel (II) sind bekannt (vergleiche DE-PS 23 24 010 [LeA 14 971] und DE-OS 23 33 354 [LeA 15148])und können nach den dort angegebenen Verfahren in üblicher Art und Weise durch Reduktion der entsprechenden Keto-Derivate erhalten werden.

Das außerdem für das erfindungsgemäße Verfahren als Ausgangsstoff zu verwendende N-(2,3-Dihydro-2,2-dimethyl-benzofuran-7-oxy-carbonyl)-N-methyl-carbamidsäurehalogenid der Formel (III) ist ebenfalls bekannt (vergleiche DE-OS 21 42 496 [LeA 13 913] und DE-OS 28 20 360 [LeA 18 831]).

Als Verdünnungsmittel kommen für das erfindungsgemäße Verfahren inerte organische Lösungsmittel infrage. Hierzu gehören vorzugsweise aromatische Kohlenwasserstoffe, wie Benzol oder Toluol; halogenierte Kohlenwasserstoffe, wie Methylenchlorid oder Tetrachlorkohlenstoff; Nitrile, wie Acetonitril oder Propionitril; Ether, wie Tetrahydrofuan oder Dioxan; sowie Ester, wie Essigsäuremethylester.

Als Säurebindemittel können für das erfindungsgemäße Verfahren alle üblichen organischen und anorganischen Säurebinder eingesetzt werden. Hierzu gehören vorzugsweise tertiäre Amine, wie Triethylamin; Alkalihydroxide und Alkalicarbonate, wie Natrium- und Kaliumhydroxid.

Die Reaktionstemperaturen können beider Durchführung des erfindungsgemäßen Verfahrens in einem größeren Bereich variiert werden. im allgemeinen arbeitet man zwischen 0 und 100°C, vorzugsweise zwischen 20 und 85°C.

Bei der Durchführung des erfindungsgemäßen Verfahrens arbeitet man vorzugsweise in äquimolaren Mengen. Die Isolierung der Endprodukte erfolgt in allgemein üblicher Art und Weise.

0124804

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I.bis II. sowie IV.bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) könnnen in einfacher Weise nach üblichen Verfahren erhalten werden, so z.B. durch Lösen des Metallsalzes in Alkohol, z.B. Ethanol und Hinzufügen zur Verbindungen der Formel(I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Fungizide können die erfindungsgemäßen Wirkstoffe mit besonders gutem Erfolg zur Bekämpfung von Getreidemehltau (Erysiphe graminis), von Gurkenmehltau (Spaerotheca fuliginea) und von Reiskrankheiten, wie Pellicularia sasakii eingesetzt werden; weiterhin können die erfindungsgemäßen Wirkstoffe als Sproßfungizide gegen Rost, Septoria nodorum, Cochliobolus sativus und Pyrenophora teres sowie als Saatbeizmittel gegen die Streifenkrankheit der Gerste (Drechslera graminea) , Schneeschimmel (Fusarium nivale) und Fusarium culmorum an Getreide eingesetzt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

0124804

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Als Insektizide können die erfindungsgemäßen Wirkstoffe mit besonders gutem Erfolg zur Bekämpfung von Doralis fabae und von Phaedon-Larven eingesetzt werden. Außerdem ist die gute bodeninsektizide und wurzelsystemische Wirkung hervorzuheben.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Gycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

0124804

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,2 %, am Wirkungsort erforderlich.
Auch beim Einsatz der erfindungsgemäßen Stoffe als Insektizide kann der Wirkstoffgehalt der aus den handelsüblichen Formulierungen bereiteten Anwendungsformen in weiten Bereich variieren. Die Wirkstoffkonzentration der Anwendungsformen kann von 0,0000001 bis zu 100 % Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten (mit Kalk gestrichenen) Unterlagen aus.

Die Herstellung und die Verwendung der erfindungsgemäßen Stoffe werden durch die folgenden Beispiele veranschaulicht.

## Herstellungsbeispiele

### Beispiel 1

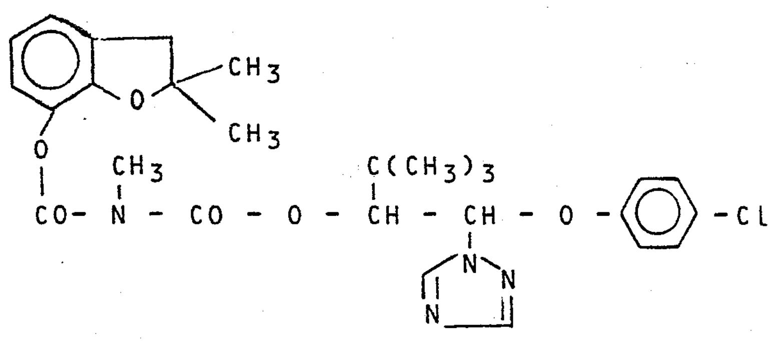

5,9g (0,017 Mol) 1-(4-Chlorphenoxy)-3,3-dimethyl-1-(1,2,4-triazol-1-yl)-2-butanol und 4,8g (0,017 Mol) N-(2,3-Dihydro-2,2-dimethyl-benzofuran-7-oxy-carbonyl)-N-methyl-carbamidsäurechlorid werden in 50 ml Toluol suspendiert und mit 2,4 ml (0,017 Mol) Triethylamin tropfenweise unter Rühren versetzt. Man läßt das Reaktionsgemisch über Nacht rühren und filtriert anschließend vom Niederschlag ab. Das Filtrat wird mit Wasser gewaschen, über Natriumsulfat getrocknet und eingeengt. Man erhält 8g (87 % der Theorie) 1-(4-Chlorphenoxy)-3,3-dimethyl-2-[N-(2,3-dihydro-2,2-dimethyl-benzofuran-7-oxy-carbonyl)-methylcarbamoyloxy]-1-(1,2,4-triazol-1-yl)-butan vom Brechungsindex $n_D^{20}$=1,5360.

Verwendungsbeispiele

In den nachfolgenden Beispielen werden die nachstehend angegebenen Verbindungen als Vergleichssubstanzen eingesetzt:

(A) 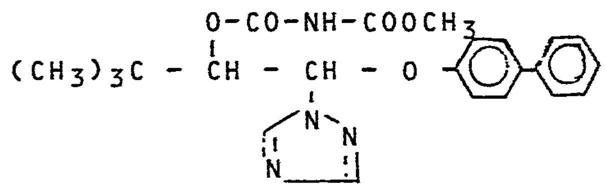

(B) 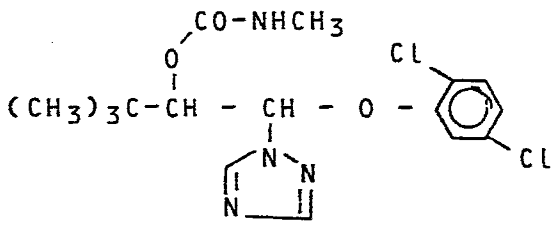

(C) 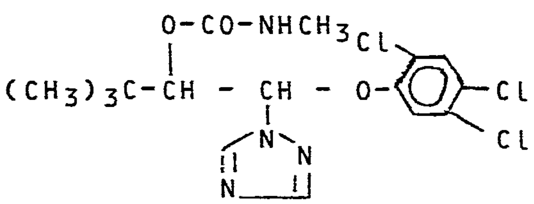

(D) 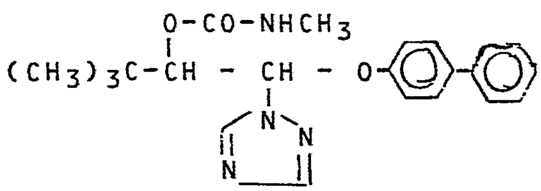

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1.

Beispiel B

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1.

Beispiel C

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1.

Beispiel D

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß folgender Herstellungsbeispiele: 1.

Beispiel E

Doralis-Test (systemische Wirkung)

Lösungsmittel: 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung werden Bohnenpflanzen (Vicia faba), die stark von der schwarzen Bohnenlaus (Doralis fabae) befallen sind, angegossen, so daß die Wirkstoffzubereitung in den Boden eindringt, ohne die Blätter der Bohnenpflanzen zu benetzen. Der Wirkstoff wird von den Bohnenpflanzen aus dem Boden aufgenommen und gelangt so zu den befallenen Blättern.

Nach der angegebenen Zeit wird die Abtötung in % bestimmt. Dabei bedeutet 100 %, daß alle Blattläuse abgetötet wurden.

Bei diesem Test zeigen z.B. die folgenden Verbindungen der Herstellungsbeispiele überlegene Wirksamkeit gegenüber dem Stand der Technik: 1.

Beispiel F

Phaedon-Larven-Test

Lösungsmittel: 3 Gewichtsteile Dimethylformamid
Emulgator: 1 Gewichtsteil Alkylarylpolyglkolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0 % bedeutet, daß keien Käfer-Larven abgetötet wurden.

Bei diesem Test zeigen z.B. die folgenden Verbindungen der Herstellungsbeispiele überlegene Wirksamkeit gegenüber dem Stand der Technik: 1.

0124804

Patentansprüche

1. Carbamoylierte Azolylalkyl-phenoxy-carbinole der allgemeinen Formel

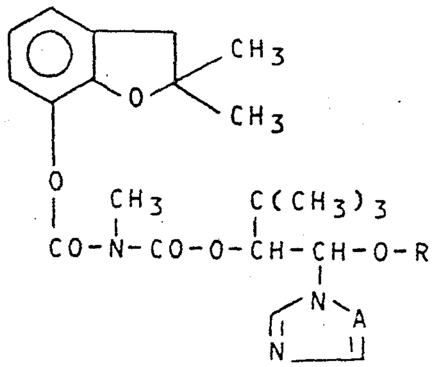

(I)

in welcher

A für ein Stickstoffatom oder die CH-Gruppe steht und

R für gegebenenfalls substituiertes Phenyl steht.

2. Verbindungen der Formel (I) in Anspruch 1, wobei

A für ein Stickstoffatom oder die CH-Gruppe steht, und

R für gegebenenfalls einfach oder zweifach, gleich oder verschieden substituiertes Phenyl steht, wobei als Substituenten genannt seien: Halogen, Alkyl mit 1 bis 4 Kohlenstoffatomen und gegebenenfalls durch Halogen substituiertes Phenyl.

3. Verbindungen der Formel (I) in Anspruch 1, wobei

A für ein Stickstoffatom oder die CH-Gruppe steht und

R für durch Fluor, Chlor, Methyl oder Phenyl substituiertes Phenyl steht.

4. Verfahren zur Herstellung von carbamoylierten Azolyl-alkyl-phenoxy-carbonolen der allgemeinen Formel

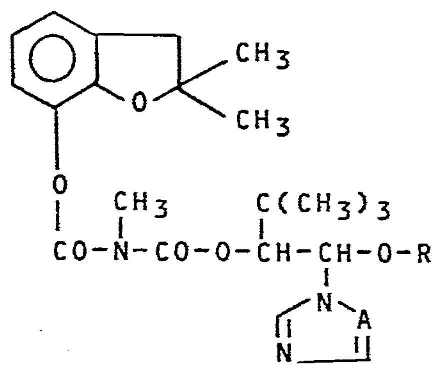

(I)

in welcher

A für ein Stickstoffatom oder die CH-Gruppe steht und

R für gegebenenfalls substituiertes Phenyl steht,

dadurch gekennzeichnet, daß man 1-Azolyl-3,3-dimethyl-1-phenoxy-2-butanole der Formel

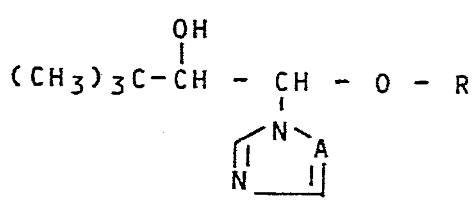

(II)

in welcher

A und R die oben angegebene Bedeutung haben,

mit N-(2,3-Dihydro-2,2-dimethyl-benzofuran-7-oxy-carbonyl)-N-methyl-carbamidsäurehalogenide der Formel

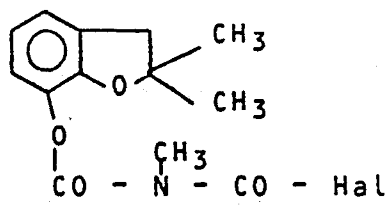

(III)

in welcher
Hal für Halogen steht,

in Gegenwart eines Verdünnungsmittels und in Gegenwart eines Säurebindemittels umsetzt.

5. Fungizide und insektizide Mittel, gekennzeichnet durch einen Gehalt an mindestens einem carbamoylierten Azolylalkyl-phenoxy-carbinol der Formel (I) in Ansprüchen 1 und 4.

6. Pflanzenschutzmittel, gekennzeichnet durch einen Gehalt an mindestens einem carbamoylierten Azolylalkyl-phenoxy-carbinol der Formel (I) in Ansprüchen 1 und 4.

0124804

7. Verfahren zur Bekämpfung von Pilzen und Insekten, dadurch gekennzeichnet, daß man carbamoylierte Azolyl-alkyl-phenoxy-carbinole der Formel (I) in Ansprüchen 1 und 4 auf Pilze oder Insekten oder ihren Lebensraum einwirken läßt.

8. Verwendung von carbamoylierten Azolylalkyl-phenoxycarbinolen der Formel (I) in Ansprüchen 1 und 4 zur Bekämpfung von Pilzen oder Insekten.

9. Verwendung von carbamoylierten Azolylalkyl-phenoxycarbinolen der Formel (I) in Ansprüchen 1 und 4 als Pflanzenschutzmittel.

10. Verfahren zur Herstellung von Pflanzenschutzmitteln, dadurch gekennzeichnet, daß man carbamoylierte Azolyl-alkyl-phenoxy-carbinole der Formel (I) in Ansprüchen 1 und 4 mit Streckmitteln und/oder oberflächenaktiven Mitteln vermischt.